(19) Europäisches Patentamt
European Patent Office
Office européen des brevets

(11) Publication number: 0 016 167
B1

(12) **EUROPEAN PATENT SPECIFICATION**

(45) Date of publication of patent specification: **26.09.84**

(51) Int. Cl.$^3$: **C 08 G 63/62,** C 07 C 37/20, C 07 C 39/06

(21) Application number: **79900922.0**

(22) Date of filing: **30.07.79**

(86) International application number: **PCT/US79/00547**

(87) International publication number: **WO 80/00348 06.03.80 Gazette 80/05**

(54) **POLYCARBONATE COMPOSITIONS HAVING IMPROVED BARRIER PROPERTIES.**

(30) Priority: **31.07.78 US 929255**

(43) Date of publication of application: **01.10.80 Bulletin 80/20**

(45) Publication of the grant of the patent: **26.09.84 Bulletin 84/39**

(84) Designated Contracting States: **DE FR GB NL**

(56) References cited:
**US-A-2 936 272**
**US-A-3 367 980**
**US-A-3 398 120**
**US-A-3 422 065**

**A CHEMICAL ABSTRACTS, VOLUME 64, NO. 4 ISSUED 14 FEBRUARY 1966, ABST. NO. 6832d, SANDLER ET AL.**

**The file contains technical information submitted after the application was filed and not included in this specification**

(73) Proprietor: **GENERAL ELECTRIC COMPANY**
**1 River Road**
**Schenectady New York 12305 (US)**

(72) Inventor: **MARK, Victor**
**701 Marigold Court**
**Evansville, IN 47712 (US)**
Inventor: **HEDGES, Charles Vernon**
**R.R. 3, Box 268**
**Mt. Vernon, IN 47620 (US)**

(74) Representative: **Catherine, Alain et al**
**GETSCO 42, avenue Montaigne**
**F-75008 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0016 167 B1**

## Description

This invention relates to high molecular weight aromatic polycarbonate compositions having improved barrier properties; i.e., low water vapor transmission and low gas permeability.

Background Art

Polycarbonate polymers are known as being excellent molding materials since products made therefrom exhibit such properties as high impact strength, toughness, high transparency, wide temperature limits (high impact resistance below —60°C and a UL thermal endurance ration of 115°C with impact), good dimensional stability, good creep resistance and the like.

Dimethylated aromatic polycarbonates are known as disclosed in U.S. Patent 3,028,365. Tetramethylated aromatic polycarbonates are also known and thetir high heat distortion properties have been recognized as disclosed in U.S. Patent 3,879,384. It would be desirable to add to this list of properties those of low water vapor transmission and low gas permeability to enable the aromatic polycarbonates to be used to form containers and film wraps for foods, beverages, cosmetics, and the like. In particular, food and beverage containers made from aromatic polycarbonates having these added barrer properties would be more economical as they would be capable of reuse and would thus also help reduce the impact of environmental waste occasioned by broken glass and discarded, non-reusable containers.

U.S. Patent 3,442,065 describes polycarbonates that are either homopolymers of a bis (hydroxyphenyl)-cycylododecane or copolymers of such bisphenols with dihydroxy compounds. These polymers are said to have good barrier properties. However, there is no suggestion that the copolymerizable dihydroxy compounds contribute to the special properties indicated, especially to the reduced permeability to steam. On the contrary, since among the listed dihydroxy compounds there are at least two, namely, bisphenol-A and 1,1-bis(4-hydroxy-phenyl)-cyclohexane, which do not show good barrier properties, it is reasonable to conclude that the special properties of the polycarbonates of this patent must be attributed to the presence of the bis(hydroxyphenyl)-cyclododecane alone.

Summary of the Invention

It has now been found that mono- and di-alkyl substituted, high molecular weight aromatic polycarbonates can be obtained that exhibit improved water vapor transmission and gas barrier properties, as compared to non-alkylated aromatic polycarbonates.

It has been further surprisingly found that mono- and dialkylated aromatic polycarbonates exhibit far superior barrier properties than either non-alkylated or tetramethylated aromatic polycarbonates.

The alkylated aromatic polycarbonates of this invention can be prepared by known techniques using appropriate monomers. The monomers that can be employed can be represented by the following general formula:

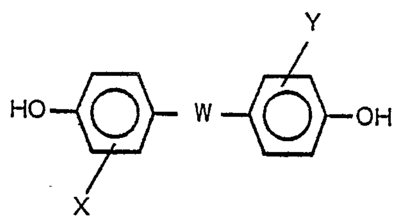

(I)

wherein X and Y can each independently be selected from hydrogen or the methyl group, with the proviso that at least either X or Y is a methyl group; and W is selected from:

(a)

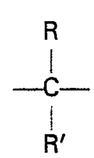

wherein R and R′ are $C_{1-2}$ alkyl with the proviso that, when both R and R′ are each $CH_3$, X and Y are not symmetrically substituting methyl groups, and:

(b) the cyclohexylidene group.

Typical of some of the monomers that can be employed in this invention are 2,2-(4-hydroxyphenyl) (4-hydroxy-3-methylphenyl)propane, 1,1-bis(4-hydroxy-3-methylphenyl)cyclohexane, and the like.

The high molecular weight aromatic polycarbonates generally have an intrinsic viscosity (IV) of about 0.40—1.0 dl/g as measured in methylene chloride at 25°C. These high molecular weight aromatic polycarbonates can be typically prepared by reacting a monomer with a carbonate precursor.

The carbonate precursor used can be either a carbonyl halide, a carbonate ester or a haloformate. The carbonyl halides can be carbonyl bromide, carbonyl chloride and mixtures thereof. The carbonate esters can be diphenyl carbonate, di-(halophenyl) carbonates such as di-(chlorophenyl) carbonate, di-(bromophenyl) carbonate, di-(trichlorophenyl) carbonate, di-(tribromophenyl) carbonate, etc., di-(alkylphenyl) carbonates such as di (tolyl) carbonate, etc., di-(naphthyl) carbonate, di-(chloronaphthyl) carbonate, phenyl tolyl carbonate, chlorophenyl chloronaphthyl carbonate, etc., or mixtures thereof. The haloformates that can be used include bis-haloformates of dihydric phenols (bischloroformates of hydroquinone, etc.) or glycols (bishaloformates of ethylene glycol, neopentyl glycol, polyethylene glycol, etc.) as well as haloformates of the monomers described above in formula I. While other carbonate precursors will occur to those skilled in the art, carbonyl chloride, also known as phosgene, is preferred.

Molecular weight regulators, acid acceptors and catalysts can also be used in obtaining the aromatic polycarbonates of this invention. The useful molecular weight regulators include monohydric phenols such as phenol, chroman-I, paratertiarybutyl-phenol, parabromophenol, primary and secondary amines, etc. Preferably, phenol is employed as the molecular weight regulator.

A suitable acid acceptor can be either an organic or an inorganic acid acceptor. A suitable organic acid acceptor is a tertiary amine such as pyridine, triethylamine, dimethylaniline, tributylamine, etc. The inorganic acid acceptor can be either a hydroxide, a carbonate, a bicarbonate, or a phosphate of an alkali or alkaline earth metal.

The catalysts which can be employed are those that typically aid the polymerization of the monomers with phosgene. Suitable catalysts include tertiary amines such as triethylamine, tripropylamine, N,N-dimethylaniline, quaternary ammonium compounds such as, for example, tetraethylammonium bromide, cetyl triethyl ammonium bromide, tetra-n-heptyl-ammonium iodide, tetra-n-propyl ammonium bromide, tetramethylammonium chloride, tetramethyl ammonium hydroxide, tetra-n-butyl ammonium iodide, benzyl-trimethyl ammonium chloride and quaternary phosphonium compounds such as, for example, n-butyltriphenyl phosphonium bromide and methyltriphenyl phosphonium bromide.

These polyfunctional aromatic compounds contain at least three functional groups which are carboxyl, carboxylic anhydride, haloformyl, or mixtures thereof. Illustrative polyfunctional aromatic compounds which can be employed include trimellitic anhydride, trimellitic acid, trimellityl trichloride, 4-chloroformyl phthalic anhydride, pyromellitic acid, pyromellitic dianhydride, mellitic acid, mellitic anhydride, trimesic acid, benzophenonetetracarboxylic acid, benzophenonetetracarboxylic anhydride, and the like. The preferred polyfunctional aromatic compounds are trimellitic anhydride and trimellitic acid or their acid halide derivatives.

Accordingly, the halogen-free, high molecular weight aromatic polycarbonates or copolycarbonates of the invention have an intrinsic viscosity of 0.40—1.0 dl/g in methylene chloride at 25°C and consist of recurring units of formula (I)

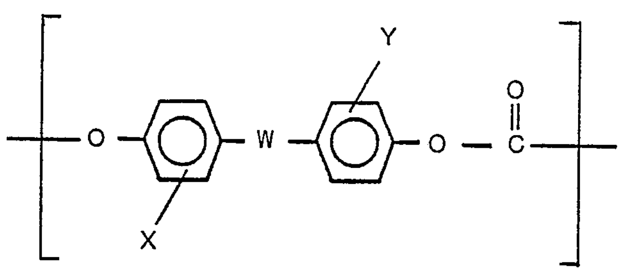

(1)

or both units of formula (1) and formula (2):

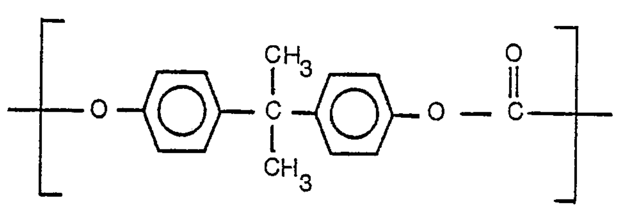

(2)

wherein the units of formula (2) are presrent in an amount up to 45 weight % of the total weight of units of formula (1) and formula (2), wherein X and Y are independently selected from hydrogen and the methyl group, with the proviso that at least either X or Y is a methyl group, and W is selected from:

a) 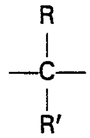

wherein R and R′ are $C_{1-2}$ alkyl, with the proviso that when both R and R′ are methyl, X and Y are not symmetrically substituting methyl groups, and:

b) the cyclohexylidene group

Preferred Embodiment of the Invention

The following examples are set forth to more fully and clearly illustrate the present invention and are intended to be, and should be construed as being, exemplary and not limitative of the invention. Unless otherwise stated, all parts and percentages are by weight.

The barrier properties for each of the ensuing examples were determined using Modern Controls, Inc. instruments. Water vapor transmission rate (WVTR) measurements were obtained on an IRD—2C instrument persuant to ASTM F—372—73; carbon dioxide data ($CO_2TR$) were obtained using a Permatran-C instrument; and, oxygen transmission rates $O_2TR$) were determined using an OX—TRAN 100 instrument. The methods used to obtain WVTR and $CO_2TR$ data are based on infrared analysis whereas the $O_2TR$ measurements are based on a coulometric method. The WVTR measurements are expressed in grams/24 hrs./645.16 $cm^2$/2.54 x $10^{-2}$ mm at 37.8°C and 90% relative humidity (RH) whereas those of $CO_2TR$ and $O_2TR$ are expressed in cc/24 hrs./645.16 $cm^2$/2.54 x $10^{-2}$ mm/101.325 Pa.

*Preparation of 4,4′-cyclohexylidenedi-o-cresol(CDC)*

To a 22 l. three-necked flask, equipped with stirrer, subsurface gas inlet tube, thermometer and reflux condenser was charged 2180 g (22.2 moles) of cyclohexanone and 12000 g (111 moles) of o-cresol and to the resulting solution was introduced subsurface HCl gas with good stirring. A mildly exothermic reaction ensued; by external cooling, the temperature was not allowed to exceed 55°—60°C. The introduction of HCl was continued until blow-through was observed, when it was stopped. The progress of the reaction was followed by ir (infrared) spectroscopy, which indicated that in ca. 1.5—2 hrs. all of the cyclohexanone was consumed, by the disappearance of the carbonyl band at 1710 $cm^{-1}$. Crystals started to come out of the warm solution in an additional 1—2 hours, which was facilitated and completed by cooling and stirring.

After the separation of crystals was complete, the reaction mixture was filtered and the filter cake was rinsed on the funnel with methylene chloride, the crystals were then slurried up in methylene chloride, filtered and the cake rinsed again. The crude crystals weight 4285 g on 76.8% of theory. The cresolic mother liquor typically contained 270 g of the p,p′-product whereas the methylene chloride mother liquors contained an additional 119 g of product, bringing the total conversion of the p,p′-isomer to 83%.

Recrystallization of the 4285 g of crude filtercake, which had an assay of 98.5% and a mp. of 184—7°C, by dissolution in 9.5 l. or refluxing methanol and subsequent addition of 2.8 l. of water, followed by cooling, deposited colourless crystals that, after filtration and airdrying, weighed 3515 g (82% of the crude), and had an assay of 99.8—99.9%, mp. 188—189°C.

Example 1

Into a mixture of 74.1 parts of pure 4,4′-cyclohexylidenedi-o-cresol (CDC) (mp 188—189°C; 0.25 parts mole), 300 parts water, 300 parts methylene chloride, 0,47 parts phenol and 0.5 parts triethylamine were introduced, at ambient temperature, 30 parts phosgene over a period of 30 minutes while maintaining the pH of the two-phase system at about 11; i.e., pH 10—12.5, by simultaneously adding a 25% aqueous sodium hydroxide solution. At the end of the addition period, the pH of the aqueous phase was 11.7 and the CDC content of this phase was less than 1 part per million (ppm) as determined by ultraviolet analysis.

The methylene chloride phase was separated from the aqueous phase, washed with an excess of dilute (0.01N) aqueous HCl and then washed three times with deionized water. The polymer was precipitated by adding the neutral and salt-free methylene chloride solutions to an excess of methanol and filtering off the white polymer which was dried at 95°C. The resultant, pure CDC-polycarbonate had an intrinsic viscosity (IV) in methylene chloride at 25°C of 0.554 dl/g. Its barrier properties are set forth in the Table following the Examples.

The intrinsic viscosity values mentioned hereinafter were also measured in methylene chloride at 25°C.

Control Example 1

The procedure of Example 1 was repeated except that 4,4′-isopropylidenediphenol, (BPA), was substituted, in equivalent amounts, for CDC. The pure BPA-polycarbonate had an IV of 0.560 dl/g. Its barrier properties are listed in the Table.

Control Example 2

Following the procedure of Example 3 of the above-mentioned U.S. Patent 3,879,384, a tetramethylated polycarbonate was prepared from 2,2-bis(3,5-dimethyl-4-hydroxyphenyl)propane. Its barrier properties are listed in the Table.

Example 2

The procedure of Example 1 was repeated, except that an equivalent amount of a 75 weight % C D C/25 weight % BPA mixture was used in place of only CDC. A copolycarbonate was obtained having an IV of 0.55 dl/g and that yielded colourless, transparent moldings or film. It barrier properties are listed in the Table.

Example 3

The procedure of Example 1 was repeated, except that an equivalent amount of a 55 weight % C D C/45 weight % BPA mixture was used in place of CDC. The resultant copolycarbonate, which yielded tough, transparent test objects and films, had an IV of 0.54 dl/g. Its barrier properties are listed in the Table.

*Preparation of 4,4′-cyclohexylidenediphenol (CDP)*

The above-described procedure for the preparation of CDC was repeated, except that 10.500 g (111.6 moles) of phenol was substituted for o-cresol. The crude crystals that separated out were rinsed with methylene chloride; after airdrying they weighed 5170 g or 87% of theoretical. Recrystallization from methanol-water yielded pure CDP, mp 188—189°C, that was 99.8% pure by gas chromatography.

*Preparation of 4,4′-(1-methylpropylidene)bis-o-Cresol (MPC)*

The above-described procedure for the preparation of CDC was repeated, except that 800 g (11.1 moles) of 2-butanone was substituted for acetone and the reaction temperature was kept at or below 50°C. Purification was effected by extracting the solid distillation residue twice with methylene chloride, which left behind the 4,4′-isomer as white crystals, mp 144°—146°C, 99.1% purity and 1840 g or 61.3% yield. This monomer, MPC, is believed to be novel.

*Preparation of 4,4′-(1-ethylpropylidene)bis-o-cresol (EPC)*

The above-described procedure for the preparation of CEC, was repeated, except that 956 g (11.1 moles) of 3-pentanone was substituted for acetone. EPC was obtained in 99.2 purity, after recrystallization from cyclohexane, with a melting point of 119—120°C.

*Preparation of 2-methyl-4,4′-(1-methylidene) bisphenol (MMEP)*

Anhydrous HCl was introduced, with adequate cooling, into a mixture of 134 g (1.0 mole) of freshly prepared 4-isopropenyl phenol (made by thermally cracking BPA in the presence of catalytic amounts of sodium hydroxide and separating it from the co-product phenol by fractional vacuum (distillation) and 540 g (5.0 moles) of o-cresol, while maintaining the temperature of the ensuing exothermic reaction below 50°C. After 4 hours, the red colored reaction mixture was stripped of HCl and excess cresol under water aspirator vacuum and the resultant straw colored melt was purified by recrystallization from cyclohexane. The 98.7% pure MMEP had a melting point of 112°—113°C. This monomer, MMEP, is a monomethyl bisphenol-A.

Examples 4—5

The preparation of polycarbonates by the procedure of Example 1 was repeated by substituting equivalent amounts of the following diphenols for CDC:

Example 4:

MPC (4,4′-(1-methylpropylidene)bis-o-cresol)

Example 5:

EPC (4,4′-(1-ethylpropylidene)bis-o-cresol)

The barrier properties of the resultant polycarbonates are listed in the Table.

Examples 6—8

The preparation of copolycarbonates with BPA by the procedure of Example 3 was repeated by substituting equivalent amounts of the following diphenols for CDC:

Reference Example

CDP (4,4′-cyclohexylidene-diphenol)

Example 6:

MPC (4,4′-(1-methylpropylidene)bis-o-cresol)

Example 7:

EPC (4,4′-1-ethylpropylidene)bis-o-cresol)

Example 8:

MMEP (2-methyl-4,4′-(1-methylethylidene bisphenol)

The barrier properties of the resultant copolycarbonates are listed in the Table wherein the Reference Example, the BPA-polycarbonate, is used as the comparative standard.

Some of the test specimens from which the barrier properties were obtained were made by extruding the polycarbonates and copolycarbonates in an extruder operated at about 265°C and, comminuting the extrudate into pellets. Thereafter, the pellets were compression molded into films having an average thickness of 0.254 mm. Other test specimens were obtained by film casting the polycarbonates and copolycarbonates directly from a methylene chloride solution to provide film specimens also having an average thickness of about 0.254 mm.

TABLE

*Barrier Properties of Polycarbonates and Copolycarbonates*

| Example | IV,dl/g | WVTR | $CO_2TR$ | $O_2TR$ |
|---|---|---|---|---|
| 1 | 0.554 | 0.7 | 20.4 | 10.2 |
| control 1 | 0.560 | 10.0 | 875 | 200 |
| control 2 | — | 8.3 | — | — |
| 2 | 0.550 | 1.6 | 148 | 34 |
| 3 | 0.540 | 2.7 | — | 56 |
| 4 | 0.526 | 2.0 | — | 34 |
| 5 | 0.555 | 1.4 | — | 28 |
| Reference | 0.547 | 6.2 | — | 110 |
| 6 | 0.552 | 5.5 | — | 140 |
| 7 | 0.538 | 5.2 | — | 132 |
| 8 | 0.544 | 5.6 | — | — |

A comparison of the measured values of the water vapor transmission rates for the polycarbonate derived from bisphenol-A (control Example 1) and tetramethylated BPA (Control Example 2) with the values measured for the polycarbonates derived from the monomethylated (Example 8) homolog of bisphenol-A, shows that the mono-methylated polycarbonates have surprisingly superior barrier properties, as is dramatically illustrated in the following list.

| Monomer | WVTR | % decrease* |
|---|---|---|
| BPA | 10 | 0 |
| monomethyl BPA | 5.6 | 44 |
| tetramethyl BPA | 8.3 | 17 |

*with respect to BPA polycarbonate

**Claims**

1. A halogen-free, high molecular weight aromatic polycarbonate or copolycarbonate having an intrinsic viscosity of 0.40—1.0 dl/g in methylene chloride at 25°C and consisting of recurring units of formula (1)

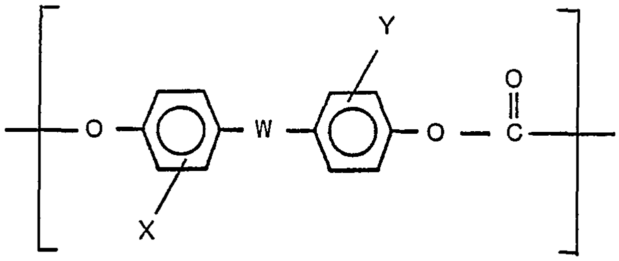

(1)

or both units of formula (1) and formula (2):

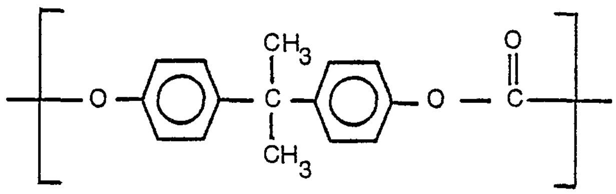

(2)

wherein the units of formula (2) are present in an amount up to 45 weight % of the total weight of units of formula (1) and formula (2), wherein X and Y are indendently selected from hydrogen and the methyl group, with the proviso that at least either X or Y is a methyl group, and W is selected from:

a) 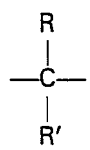

wherein R and R′ are $C_{1-2}$ alkyl, with the proviso that when both R and R′ are methyl, X and Y are not symmetrically substituting methyl groups, and;
b) the cyclohexylidene group.

2. The polycarbonate of claims 1 wherein W is cyclohexylidene, 1-methyl-propylidene, or 1-ethyl-propylidene, and X and Y are both methyl groups ortho to the respective oxygens.

3. The monomer, 4,4′-(1-methylpropylidene)bis-o-cresol.

4. The monomer, 4,4′-(-1-ethylpropylidene)bis-o-cresol.

## Patentansprüche

1. Halogenfreies, hochmolekulares, aromatisches Polycarbonat oder Copolycarbonat mit einer grundmolaren Viskositätszahl von 0,40 bis 1,0 dl/g in Methylenchlorid bei 25°C und bestehend aus wiederkehrenden Einheiten der Formel (1)

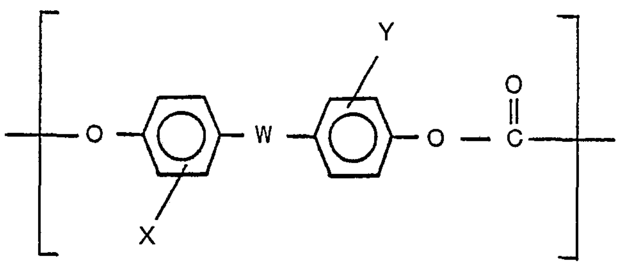

(1)

oder Einheiten der Formel (1) und der Formel (2)

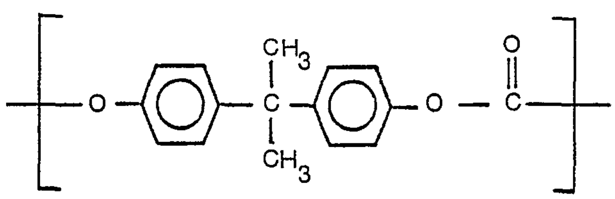

(2)

worin die Einheiten der Formel (2) in einer Menge bis zu 45 Gew.-% des Gesamtgewichts der Einheiten der Formel (1) und Formel (2) vorhanden sind, worin X und Y unabhängig voneinander ausgewählt sind aus Wassserstoff und der Methylgruppe, mit der Maßgabe, daß wenigstens entweder X oder Y eine Methylgruppe ist und W ausgewählt ist aus:

(a) 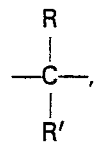

worin R und R' $C_{1-2}$-Alkylgruppen sind, mit der Maßgabe, daß dann, wenn beide R- und R'-Reste Methylgruppen sind, X und Y keine symmetrisch substituierende Methylgruppen sind, und
(b) der Cyclohexylidengruppe.

2. Polycarbonate nach Anspruch 1, worin W Cyclohexyliden, 1-Methylpropyliden oder 1-Äthylpropyliden ist und X und Y beide Methylgruppen in o-Stellung zu den jeweiligen Sauerstoffatomen sind.

3. Das Monomere 4,4'-(1-Methylpropyliden)-bis-o-kresol.

4. Das Monomere 4,4'-(1-Äthylpropyliden)-bis-o-kresol.

**Revendications**

1. Polycarbonate ou copolycarbonate aromatique de masse molaire élevée, sans halogène, ayant un indice limite de viscosité de 0,40—1.0 dl/g dans le chlorure de méthylène à 25°C et se composant de motifs récurrents de formule (1)

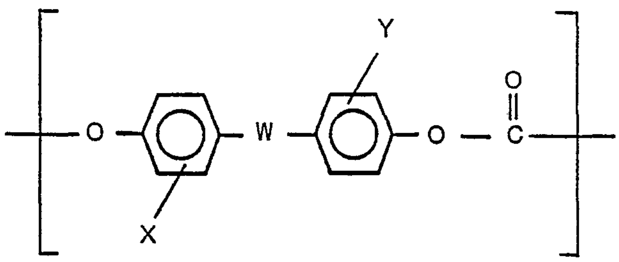

(1)

ou à la fois de motifs de formule (1) et de formule (2)

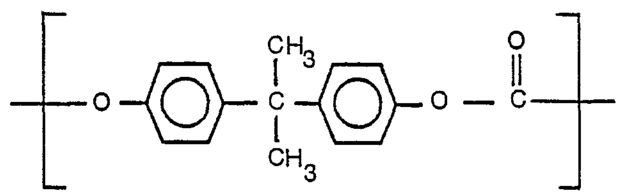

(2)

où les motifs de formule (2) sont présents en une quantité allant jusqu'à 45% en poids du poids total des motifs de formule (1) et de formule (2), où X et Y sont indépendamment choisis entre l'hydrogène et le groupe méthyle, avec la condition que au moins X ou Y soit un groupe méthyle, et W est choisi parmi:

(a) 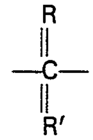

où R et R' sont des radicaux alkyles et $C_{1-2}$ avec la condition que, lorsque à la fois R et R' sont chacun en $CH_3$, X et y ne sont pas des groupes méthyles de substitutions symétriques et:
(b) le groupe cyclohexilidène.

2. Polycarbonate selon la revendication 1 dans lequel W est le groupe cyclohexylidène, méthyl-1-propylidène ou éthyl-1 propylidène, et X et Y sont tous deux des groupes méthyles en position ortho par rapport aux oxygènes respectifs.

3. Le monomère (méthyl-1 propylidène)-4,4' bis-o-crésol.

4. Le monomère (éthyl-1 propylidène)-4,4' bis-o-crésol.